# EUROPEAN PATENT SPECIFICATION

(11) **EP 1 629 812 B1**
(45) Date of publication and mention of the grant of the patent: **12.03.2008**
(21) Application number: 05425588.0
(22) Date of filing: 10.08.2005
(51) Int. Cl.: A61F 13/44

(54) **A surgical dressing with identification counterfoil**
Chirurgischer Tupfer mit einem Identifizierungkontrollabschnitt
Compresse chirurgicale avec un talon d' identification

(30) Priority: 31.08.2004 IT RN20040042
(43) Date of publication of application: 01.03.2006
(73) Proprietor: Galdenzi, Alessandro, 60019 Senigallia AN (IT); Gregorini, Mirco, 60019 Senigallia AN (IT)
(72) Inventor: Galdenzi, Alessandro, 60019 Senigallia AN (IT); Gregorini, Mirco, 60019 Senigallia AN (IT)
(74) Representative: Ghioni, Carlo Raoul Maria

(56) References cited:
- WO-A-91/04723
- WO-A-95/00098
- WO-A-98/10367
- DE-A1- 4 239 213
- DE-U1- 9 014 500
- FR-A- 2 742 655
- GB-A- 1 422 414

## Description

The present invention relates to a dressing for surgical use.

In the surgical sector, a problem with extremely serious implications is represented by the possibility of accidentally losing a foreign object, such as a surgical instrument or one of the dressings used in the operation, in the patient's body.

In the case of dressings, the probability of such an accident occurring is relatively high since the number of dressings used can sometimes be considerable, and/or because the final condition of the dressings can make them difficult to recognise The used dressings may in fact be so soaked in body fluids or be folded, crumpled or compacted by the surgeon as to make their identification and recognition, inside the patient's body and between the various organs, quite difficult.

On the other hand, it is also understandable that in the unfortunate event of a dressing remaining in the patient's body, its identification and location after the operation, perhaps even after a considerable time, would be an extremely difficult task.

Surgical dressings incorporating a strip of radio-opaque material are therefore currently used. If any dressing is lost, a radioscopic examination makes it possible to identify and locate the dressing in the patient's body.

In terms of prevention of the risk of losing the dressing, the means currently used to prevent the possibilities described above is however represented solely by counting the dressings relative to a certain operation at the start and at the end and by ascertaining that these numbers are identical.

This method - which is moreover commonly used - is nevertheless extremely restrictive and in no way reassuring.

In fact, even if the totals coincide one can never exclude the possibility with absolute certainty that a dressing has been left in the patient's body. It would in fact be sufficient to have wrongly counted one more dressing than those actually prepared or to have written down the total number of dressings increased by one with respect to the true total to invalidate the conclusion drawn from the coincidence of the compared results of the two counts and to demonstrate the definitive inefficacy of the method used at present.

There is also a certain margin of risk due to the fact that the initial and final numbers of dressings may involve different people, with the consequent possibility of errors; in counting; in communicating the number counted; and also in attributing the responsibilities involved in these acts. This may happen, for example, when an operation goes on beyond the end of a shift, when the nursing staff change over in the operating theatre.

Document FR2742655 discloses an assembled dressing for surgical use which comprises a first section being designed for surgical use and a second section being designed as a counterfoil for the first section used. The dressing is preventively assembled and during use the second section is unthreaded by simple sliding.

Document GB1422414 discloses a bundle of five swabs which is held together at one corner by a length of plastics monofilament which is in turn connected with a tape. The plastic monofilament must be broken in order to use the swabs.

The aim of the present invention is to overcome the problem described above in such a way as to allow a sure, objective count of the dressings initially prepared for a specific operation that can be repeated at any time and whenever wanted and so as to allow a rapid check with the necessary certainty that the total number of dressings present at the end of the operation actually coincides with the number of those prepared initially. An additional obvious objective is to provide a concrete contribution to the ever greater attempts at minimising operative risks.

According to the invention, this aim is achieved by a dressing as claimed in claim 1.

The technical characteristics of the invention, with reference to the above aims, are clearly described in the claims below and its advantages are apparent from the detailed description which follows, with reference to the accompanying drawings which illustrate a preferred embodiment of the invention provided merely by way of example without restricting the scope of the inventive concept, and in which:
- Figure 1 shows a perspective overall view of a dressing according to the invention shown in an initial configuration, before its use;
- Figure 2 is a perspective overall view of the dressing of Figure 1 when it is used;
- Figure 3 is a perspective overall view of a plurality of dressings according to the invention wrapped around themselves in order to acquire the typical shape of a swab.

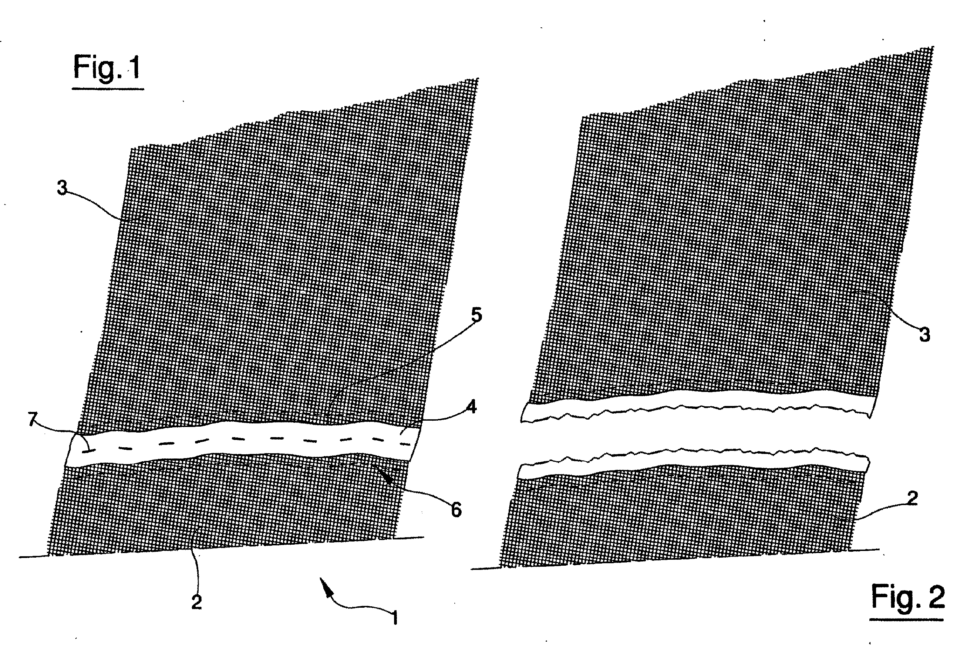

With reference to Figure 1 in the accompanying drawing, 1 indicates a surgical dressing comprising two distinct component sections 2 and 3, connected to each other by an intermediate joint 4.

The joint 4 is made by a strip of appropriate material connected to the two sections 2 and 3 by connecting means which in the accompanying figures are represented by way of example by two seams 5 and 6.

The strip which forms the joint 4 includes a precut broken line 7 designed to allow easy and even breaking of the joint 4. The joint 4 is thus breakable, making it possible to separate, by tearing, the two sections 2 and 3 as shown in Figure 2.

The first 3 of the sections forming the dressing 1 is designed for surgical use in a totally conventional way. The second 2 of the sections is instead designed to form a counterfoil for the automatic memorising of the dressings actually used thus allowing fast and sure counting, at any time and by anyone, of all the first sections 3 used during the operation and in particular to provide a very quick and absolutely certain check of the number of first sections 3 that must be recovered in order to be sure that at the end of the operation none have been left inside the patient's body.

Since the dressing 1 according to the invention makes it possible to check with absolute certainty that no dressing 1 has been left inside the patient's body, any material could, generally speaking, be used to form the joint 4, as long as it is appropriate for the use for which it is designed and allows even tearing so as to prevent even the smallest particle of foreign material from being left in the patient's body. However, for maximum safety and when it is necessary to rapidly identify, using a radioscopic method, a dressing lost in the patient's body, the strip can advantageously be made from radio-opaque material.

Such a solution would also have the advantage of having practically the same costs for the dressings described above as traditional dressings.

In the non-restricting example described above, the term dressing means a dressing which, at least for the section designed to come into contact with the patient's anatomy, is woven and is made in a conventional way from natural or artificial fibres. It is, however, clear that the same term can also be used to indicate products having the same function and made from different materials, in which, particularly the section 3 can be made for example from any other material (cellulose, non-woven fabric, etc.) as long as it is appropriate for the use for which the dressing is designed.

As regards the second section 2 designed to act as a counterfoil, it is equally evident that this does not necessarily have to be made from the same material as the first section 3. In fact, the presence of a joint 4 separating the two sections 2 and 3 makes it extremely easy to produce dressings 1 which combine different materials for the two sections 2 and 3.

If the dressings are supplied in numbered bundles with a progressive number on the second section, counting the dressings used, that is to say the dressings that must be recovered from the patient's body at the end of the operation, is completely automatic, easy, immediate and sure.

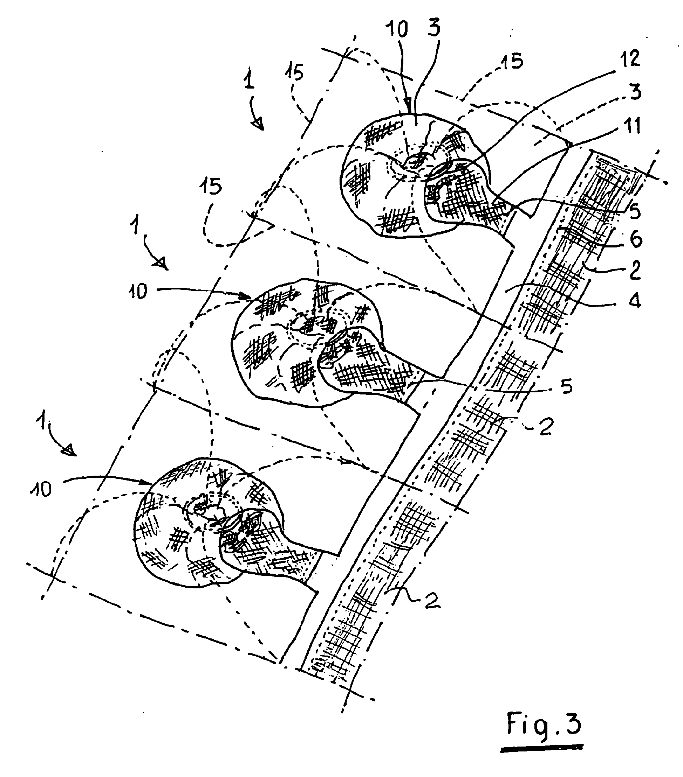

One such variation is shown in Figure 3. From the figure it can be seen that a plurality of adjacent dressings 1, preferably arranged in strips and initially flat, present their relative first sections 3, with their edges 15, that can be wrapped up so as to form, with the aid of an elastic retaining ring, large sphere-shaped swabs 10 which remain connected to their respective second sections 2 by means of a narrow bridge area 11 over the joint 4 between the first 3 and second sections 2.

More specifically, the swabs 10 are obtained: by wrapping the first section 3 of the dressing 1 around the elastic ring 12; by subsequently inserting the corners of the first section 3 of the dressing 1 in the opening of the ring 12 (as schematically shown by the broken lines in Figure 3); and finally, by filling the swab 10, that is the dressing pouch formed by the first section 3, with the edges of the section that are forcedly pushed into the pouch to fill it.

To use the swabs 10, formed in this way, it is sufficient to tear the bridge area 11 of each swab 10 from the second section 2 which thus acts as a counterfoil for the swab 10. The flap of the bridge area 11 associated with the swab 10 can obviously then also be inserted in the ring 12 if its presence outside the swab 10 is not required or is not compatible with the use of the swab 10.

## Claims

1. A dressing which comprises two distinct component sections (2,3) and a joint (4) breakably connecting the sections (2,3), a first section (3) being designed for surgical use and the second section (2) being designed to act as a counterfoil for the section (3) used, **characterised in that** the joint (4) consists of a strip positioned between and connected to the component sections (2,3) of the dressing (1), the strip having a precut line (7), designed to allow the separation by tearing of the component sections (2,3).

2. The dressing according to claim 1, **characterised in that** the joint (4) is made from radio-opaque material.

3. The dressing according to claim 1, **characterised in that** at least the first section (3) is made from woven material.

4. The dressing according to claim 1, **characterised in that** at least the first section (3) is made from non-woven material.

5. The dressing according to any of the foregoing claims, **characterised in that** the sections (2,3) are made from different materials to each other.

6. The dressing according to any of the foregoing claims, **characterised in that** at least the second section (2) bears a number.

7. The dressing, according to any of the foregoing claims, forming together with a retaining ring (12) a gauze swab (10), **characterised in that** the edges (15) of the first section (3) wrap around the ring (12) and, passing through the opening, penetrate the swab (10), a bridge area (11) of the first section (3) connecting the swab (10) to the second section (2), it being possible to separate the swab (10) from the second section (2) by breaking the joint (4) at the level of the bridge area (11).

## Patentansprüche

1. Wundverband, welcher zwei unterschiedliche Komponentenabschnitte (2, 3) enthält, sowie eine Verbindung (4), die lösbar die beiden Abschnitte (2, 3) miteinander verbindet, wobei ein erster Abschnitt (3) für chirurgische Zwecke bestimmt ist, und der zweite Abschnitt (2) dazu vorgesehen ist, als Kontrollabschnitt für den verwendeten Abschnitt (3) zu dienen, **dadurch gekennzeichnet, dass** die Verbindung (4) aus einem Streifen besteht, der zwischen den beiden Komponentenabschnitten (2, 3) des Wundverbandes (1) positioniert und an diese angeschlossen ist, wobei der Streifen eine vorgeprägte Linie (7) hat, die dazu bestimmt ist, das Trennen der Komponentenabschnitte (2, 3) durch Reissen zu erlauben.

2. Wundverband nach Patentanspruch 1, **dadurch gekennzeichnet, dass** die Verbindung (4) aus einem strahlenundurchlässigen Material hergestellt ist.

3. Wundverband nach Patentanspruch 1, **dadurch gekennzeichnet, dass** wenigstens der erste Abschnitt (3) aus einem gewebten Material hergestellt ist.

4. Wundverband nach Patentanspruch 1, **dadurch gekennzeichnet, dass** wenigstens der erste Abschnitt (3) aus einem nicht gewebten Material hergestellt ist.

5. Wundverband nach einem beliebigen der vorstehenden Patentansprüche, **dadurch gekennzeichnet, dass** die Abschnitte (2, 3) aus voneinander unterschiedlichen Materialien hergestellt sind.

6. Wundverband nach einem beliebigen der vorstehenden Patentansprüche, **dadurch gekennzeichnet, dass** wenigstens der zweite Abschnitt (2) eine Nummer trägt.

7. Wundverband nach einem beliebigen der vorstehenden Patentansprüche, welcher zusammen mit einem Haltering (12) einen Gazetupfer (10) bildet, **dadurch gekennzeichnet, dass** sich die Ränder (15) des ersten Abschnittes (3) um den Ring (12) wickeln und, indem sie durch die Öffnung gehen, in den Tupfer (10) eindringen, wobei ein Brückenbereich (11) des ersten Abschnittes (3) den Tupfer (10) mit dem zweiten Abschnitt (2) verbindet, und wobei es möglich ist, den Tupfer (10) durch Lösen der Verbindung (4) auf der Höhe des Brückenbereiches (11) von dem zweiten Abschnitt (2) zu trennen.

## Revendications

1. Compresse comprenant deux sections distinctes (2, 3) et une jointure (4) connectant de manière séparable les sections (2, 3), une première section (3) étant destinée à une utilisation chirurgicale et la seconde section (2) étant destinée à agir en tant que talon pour la section (3) utilisée, **caractérisée en ce que** la jointure (4) consiste en une bande positionnée entre les sections composantes (2, 3) de la compresses (1) en étant connectée à ces dernières, la bande ayant une ligne prédécoupée (7), destinée à permettre la séparation par déchirement des sections composantes (2, 3).

2. Compresse selon la revendication 1, **caractérisée en ce que** la jointure (4) est réalisée en un matériau radio-opaque.

3. Compresse selon la revendication 1, **caractérisée en ce qu'**au moins la première section (3) est réalisée en un matériau tissé.

4. Compresse selon la revendication 1, **caractérisée en ce qu'**au moins la première section (3) est réalisée en un matériau non-tissé.

5. Compresse selon n'importe laquelle des revendications précédentes, **caractérisée en ce que** les sections (2, 3) sont réalisées en des matériaux différents entre eux.

6. Compresse selon n'importe laquelle des revendications précédentes, **caractérisée en ce qu'**au moins la seconde section (2) supporte un nombre.

7. Compresse, selon n'importe laquelle des revendications précédentes, formant avec un anneau de retenue (12) un tampon de gaze (10), **caractérisé en ce que** les bords (15) de la première section (3) enveloppent l'anneau (12) et, en passant au travers de l'ouverture, pénètrent dans le tampon (10), une zone pont (11) de la première section (3) reliant le tampon (10) à la seconde section (2), ceci étant possible pour séparer le tampon (10) de la seconde section (2) en cassant la jointure (4) au niveau de la zone pont (11).
